# EUROPEAN PATENT APPLICATION

(11) **EP 2 677 026 A1**
(43) Date of publication of application: **25.12.2013**
(21) Application number: 12004637.0
(22) Date of filing: 20.06.2012
(51) Int. Cl.: C12N 5/071

(54) **Keratinocyte minimal growth medium and use thereof in an in vitro method for identifying compounds capable of improving skin and hair physiology**

(71) Applicant: Merz Pharma GmbH & Co. KGaA, 60318 Frankfurt (DE)
(72) Inventor: Abts, Harry, Frank, Dr., 61440 Oberursel (DE); Hengl, Thomas, Dr., rer., nat., 60437 Frankfurt am Main (DE); Schlinzig, Kim, 61440 Oberursel (DE)
(74) Representative: Ricker, Mathias

(57) **Abstract**

The present invention relates to a serum-free keratinocyte culture medium that allows keratinocytes to be cultured under growth limiting conditions. This culture medium mimics the reduced activity of keratinocytes under malnutrition in the skin and particularly of keratinocytes at the hair-follicle involved in diffuse hair-loss. In addition, the present invention relates to the use of the serum-free keratinocyte culture medium for screening for candidate compounds capable of improving skin and hair physiology and to protect the skin, in particular keratinocytes, from negative effects of UV irradiation.

## Description

### FIELD OF THE INVENTION

The present invention relates to a serum-free keratinocyte culture medium that allows keratinocytes to be cultured under growth limiting conditions. This culture medium mimics the reduced activity of keratinocytes under malnutrition in the skin and particularly of keratinocytes at the hair-follicle involved in diffuse hair-loss. In addition, the present invention relates to the use of the serum-free keratinocyte culture medium for screening for candidate compounds capable of improving skin and hair physiology and to protect the skin, in particular keratinocytes, from negative effects of UV irradiation.

### BACKGROUND OF THE INVENTION

The cultivation of epidermal keratinocytes is known since decades but suffered from major problems until 1975, when Rheinwald and Green reported the serial cultivation of pure cultures of keratinocytes from a single-cell suspension of epidermal cells (Rheinwald, J. G. & Green, H., Cell 6: 331-343 (1975)). This was achieved by growing the cells in serum-containing medium on a feeder cell layer of a specific established cell line.

Continuous modifications and refinements to culture techniques have been made since then, allowing feeder-free serial cultivation. Furthermore, alternative methods of keratinocyte culture have been developed that avoid the use of serum. Current keratinocyte culture media are usually serum-free media which contain growth promoting factors like bovine pituitary gland extract (BPE) and epidermal growth factor (EGF). BPE and EGF both stimulate cultured human keratinocytes and are essential components for sustained proliferation of keratinocyte populations in serum-free medium.

Under certain circumstances, however, this super-optimal growth environment and resulting maximum proliferation of conventional keratinocyte cell cultures is not desirable. This applies to situations of malnutrition like that anticipated at the hair-follicle in case of diffuse hair-loss. In this case, other cell cultures than the conventional cell cultures are needed that more closely resemble the situation under malnutrition.

In one report, the use of deficient media for human keratinocytes, namely a commercial growth media (KGM, GibcoBRL) without L-cysteine, L-methionine, L-histidine, D-pantothenate, bovine pituitary extract (BPE) and epithelial growth factor (EGF), was described (Hoeller et al., Cutaneous and Ocular Toxicology 25: 13-22 (2006)). However, for these media a complete block of proliferation was observed during cultivation. Furthermore, no single compound or combination of compounds was demonstrated to restore proliferation of keratinocytes in these media. Thus, the deficient cell culture media systems described by Hoeller *et al.* appear to result in cells which are "frozen" in den G₀/G₁-cell cycle without being able to re-enter proliferation status.

Thus, there is still a need for an *in vitro* cell culture system resembling limited proliferation of keratinocytes during malnutrition.

### SUMMARY OF THE INVENTION

The present invention provides a culture medium that allows keratinocytes to be cultured under growth-limiting conditions. These growth-limiting conditions mimic the reduced activity of keratinocytes under malnutrition in the skin and, in particular, of keratinocytes at the hair-follicle involved in diffuse hair-loss. The culture medium of the present invention can be used in an *in vitro* minimal growth assay system for screening for candidate compounds for improving skin and hair physiology or for protecting keratinocytes from negative effects of UV irradiation.

More specifically, the present invention provides a serum-free keratinocyte culture medium, wherein the culture medium lacks cysteine and/or thiamin and, optionally, one or more of pantothenate, folic acid and biotin, or derivatives thereof.

In addition, the present invention relates to the use of the serum-free keratinocyte culture medium of the invention for screening for candidate compounds for improving skin and hair physiology or for protecting keratinocytes from negative effects of UV irradiation.

The present invention further provides an *in vitro* method for identifying one or more compounds capable of changing the functional activity of keratinocytes under growth-limiting conditions, the method comprising:
(a) contacting keratinocytes with the serum-free keratinocyte culture medium according to the present invention and one or more test compounds;
(b) determining the functional activity of the keratinocytes at a particular time after contacting the keratinocytes with the serum-free keratinocyte culture medium;
(c) assessing the capability of the one or more test compounds to change the functional activity of the keratinocytes.

Furthermore, the present invention provides an *in vitro* method for identifying one or more compounds capable of protecting keratinocytes from negative effects of UV irradiation, the method comprising:
(a) contacting keratinocytes with the serum-free keratinocyte culture medium according to the present invention and one or more test compounds;
(b) irradiating the keratinocytes with a certain sublethal dose of UV radiation after contacting the keratinocytes with the serum-free keratinocyte culture medium for a particular time;
(c) determining a functional activity of the keratinocytes at a particular time after completion of the UV irradiation; and
(d) assessing the capacity of the one or more test compounds to change the functional activity of the keratinocytes to thereby identify one or more compounds capable of protecting keratinocytes from negative effects of UV radiation.

Preferred embodiments of the present invention are set forth in the appended claims.

The present invention may be more fully understood by reference to the following detailed description of the present invention, the examples, and the accompanying drawings.

### BRIEF DESCRIPTION OF THE DRAWINGS

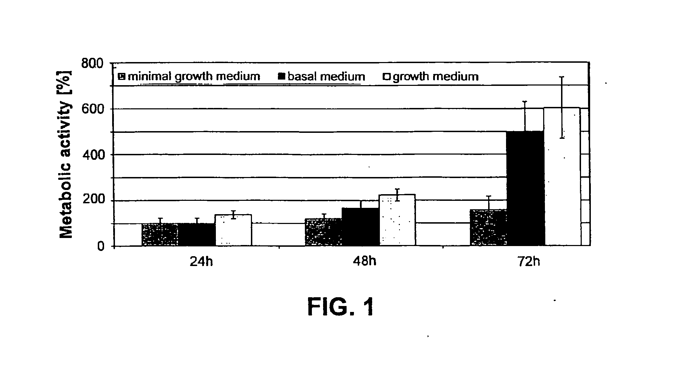

**FIG. 1** is a bar graph showing, for each of the three culture media (minimal growth medium (MGM), basal medium (BM) and growth medium (GM)), the metabolic activity at 24 h, 48 h and 72 h. The metabolic activity is shown relative to that of the minimal growth medium (MGM), which is set 100%.

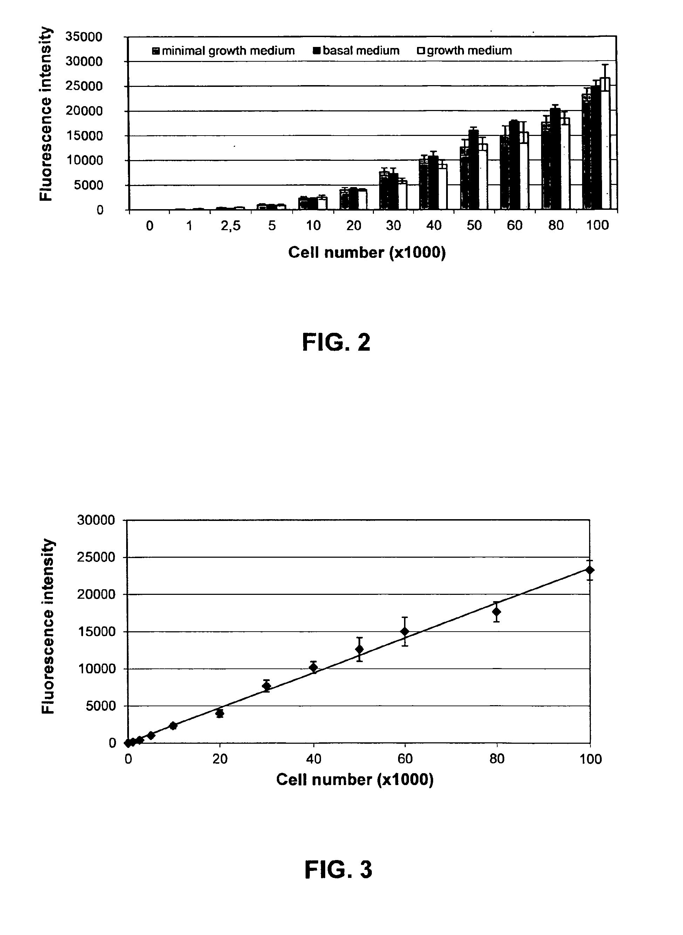

**FIG. 2** is a bar graph showing the cell number dependent metabolic activity of NHEK (normal human epidermal keratinocytes) cultivated for 15 h in minimal growth medium (MGM), basal medium (BM) and growth medium (GM).
**FIG. 3** is a diagram showing the linear regression of metabolic activity in minimal growth medium (MGM) as a function of cell number.

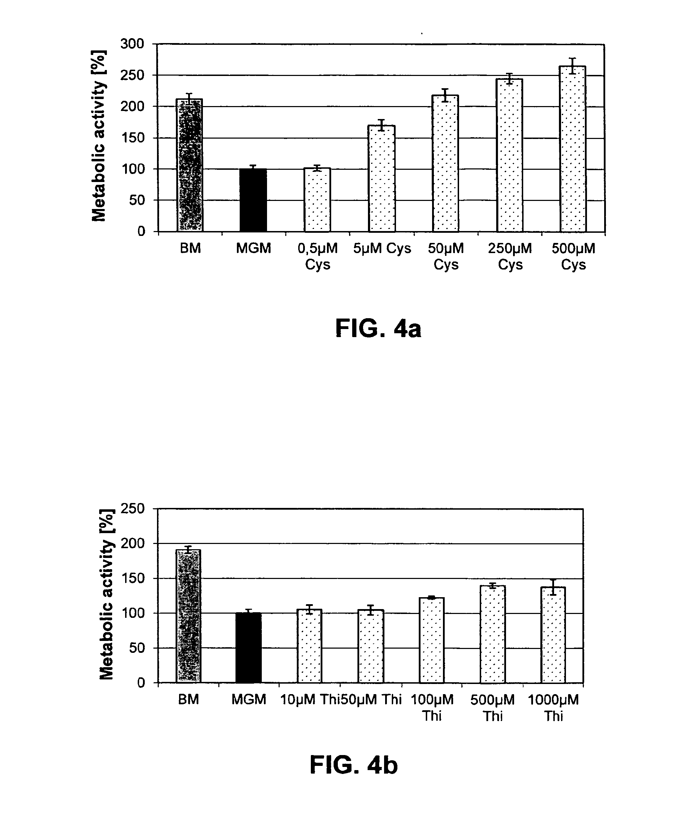

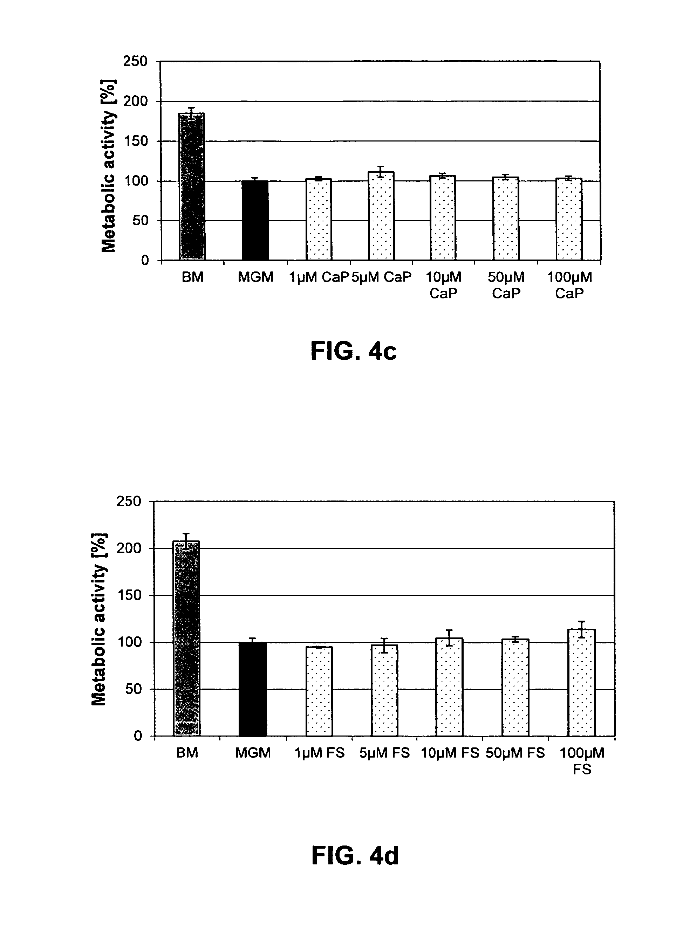

**FIG. 4** depicts different bar graphs showing the metabolic activity of NHEKs cultivated for 48 h in minimal growth medium (MGM) and increasing concentrations of (a) L-cystine, (b) thiamine, (c) pantothenate, and (d) folic acid. The metabolic is shown relative to that of the minimal growth medium (MGM), which is set 100%. For comparison, the metabolic activity of the basal medium (BM) is also shown.

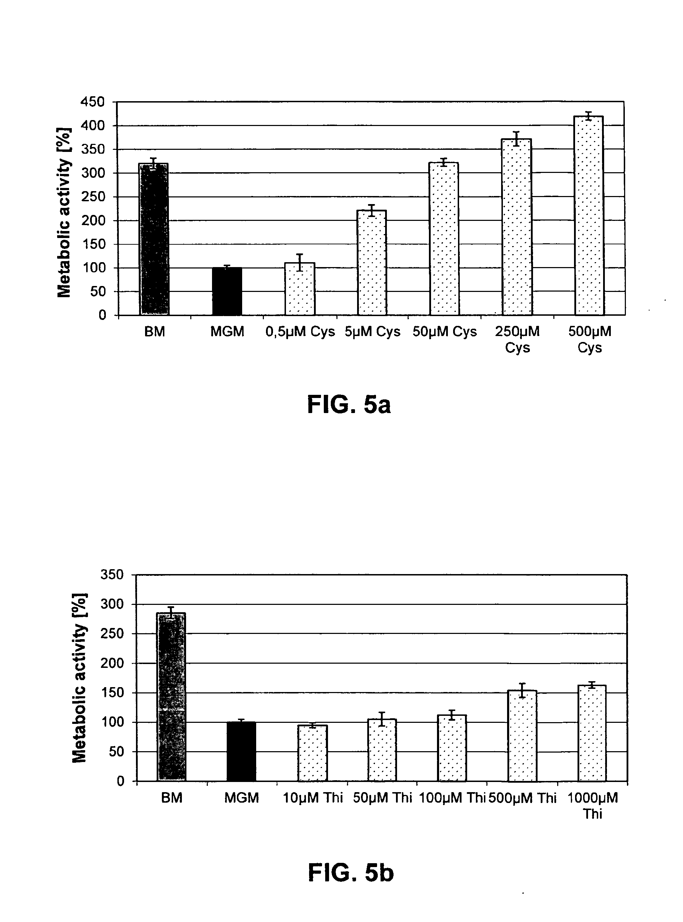

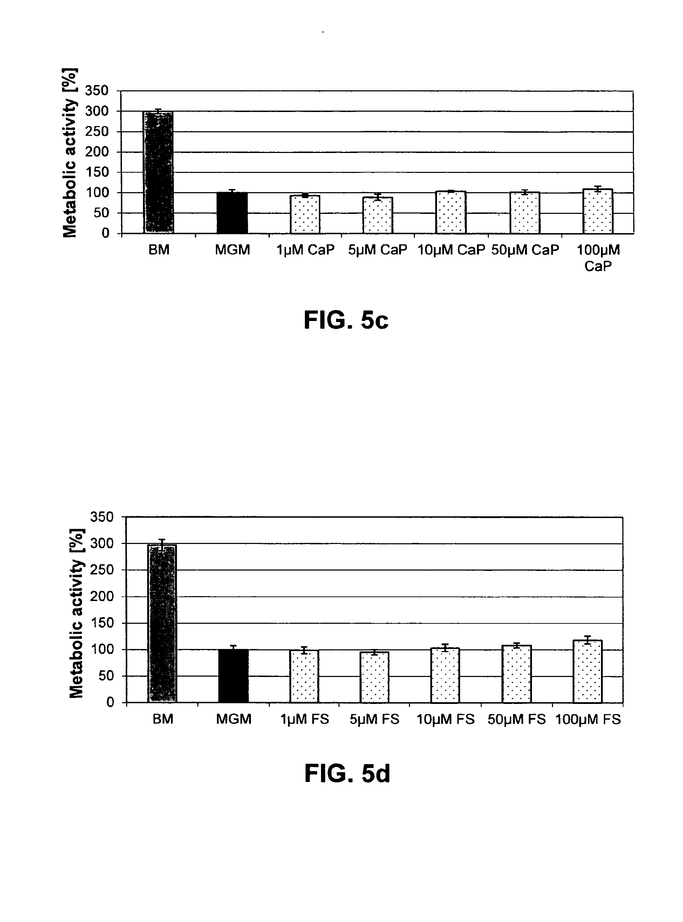

**FIG. 5** depicts different bar graphs showing the metabolic activity of NHEKs cultivated for 72 h in minimal growth medium (MGM) and increasing concentrations of (a) L-cystine (Cys), (b) thiamine (Thi), (c) calcium pantothenate (CaP), and (d) folic acid (FS). The metabolic activity is shown relative to that of the minimal growth medium (MGM), which is set 100%. For comparison, the metabolic activity of the basal medium (BM) is also shown.

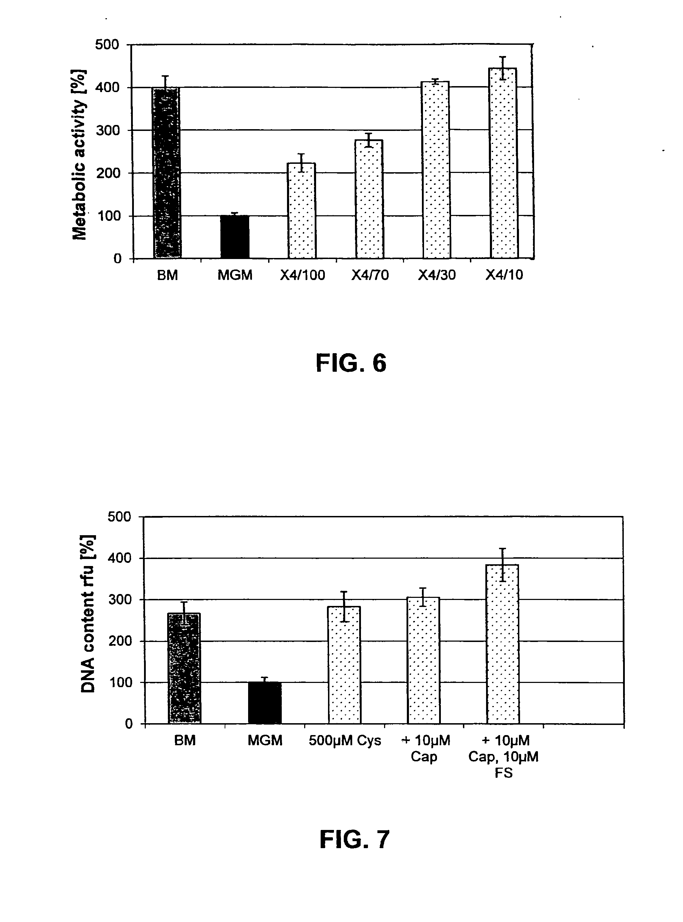

**FIG. 6** is a bar graph showing the metabolic activity of NHEKs cultivated for 72 h in basal medium (BM), minimal growth medium (MGM), and MGM + Pantogar-IC at different concentrations (X4 = Pantogar-IC = Pantogar *in vitro* correlate consisting of 500 µM L-cystine, 1000 µM thiamin, 10 µm pantothenate and 10 µm folic acid, i.e. X4/10 = 1/10 of the indicated concentrations).
**FIG. 7** is a bar graph showing the proliferation (i.e. DNA content) of NHEK cultivated in basal medium (BM), minimal growth medium (MGM) and MGM + combinations of single Pantogar-IC compounds (L-cysteine (Cys); calcium pantothenate (CaP), and folic acid (FS)).

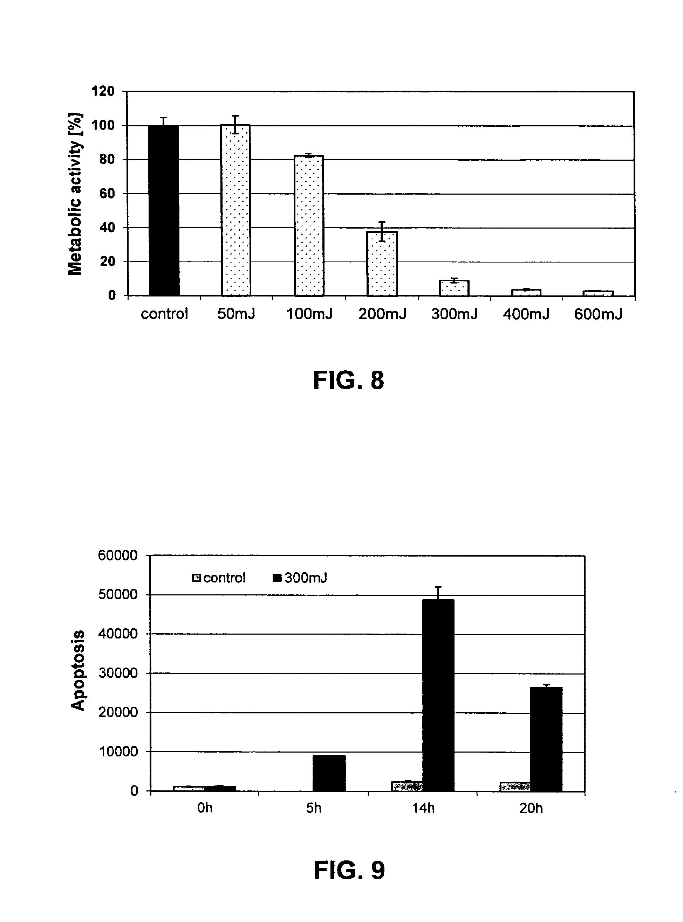

**FIG. 8** is a bar graph showing the metabolic activity of UV-irradiated NHEK in minimal growth medium (MGM) 24 h after UV-irradiation at a dose of 50 mJ, 100 mJ, 200 mJ, 300 mJ, 400 mJ and 600 mJ (based on the UV-B-part (290 nm to 315 nm)) of the used solar simulated spectrum).
**FIG. 9** is a bar graph showing the degree of apoptosis of UV-irradiated NHEK in minimal growth medium (MGM) 0 h, 5 h, 14 h and 20 h after UV irradiation at a dose of 300 mJ (UV-B).

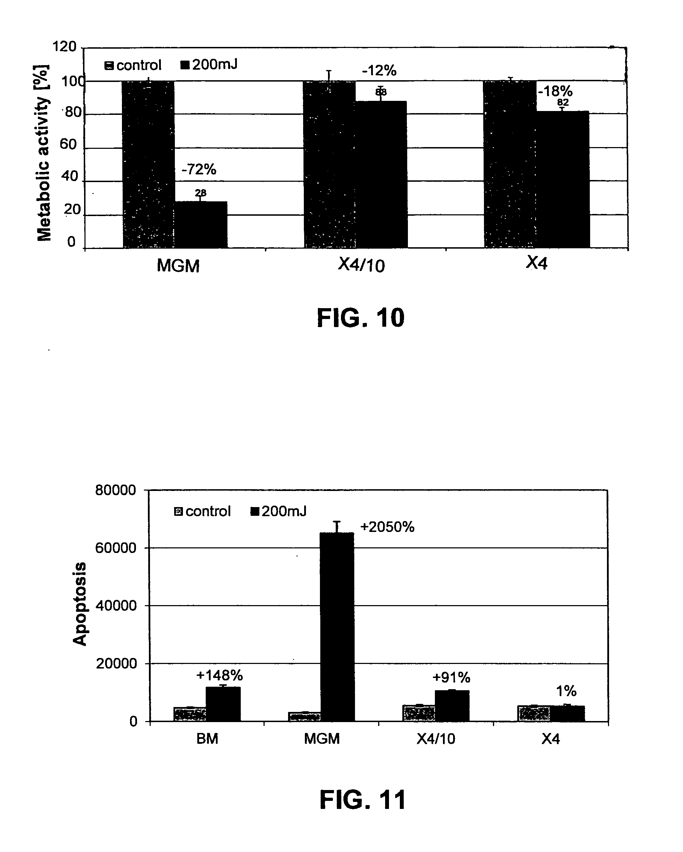

**FIG. 10** is a bar graph showing the metabolic activity of NHEK in minimal growth medium (MGM), MGM + Pantogar-IC (X4/10) and MGM + Pantogar-IC (X4), respectively, 24 h after UV irradiation at a UV-B dose of 200 mJ. The numbers -72%, -12% and -18% indicate the percentage decrease of metabolic activity compared to the metabolic activity of the respective non-UV-irradiated control, which is set 100%.
**FIG. 11** is a bar graph showing the degree of apoptosis of NHEK in basal medium (BM), minimal growth medium (MGM), MGM + Pantogar-IC (X4/10) and MGM + Pantogar-IC (X4), respectively, 14 h after UV irradiation at a UV-B dose of 200 mJ. The numbers +148%, +2050%, +91% and +1% indicate the percentage increase of apoptosis compared to apoptosis of the respective non-UV-irradiated control.

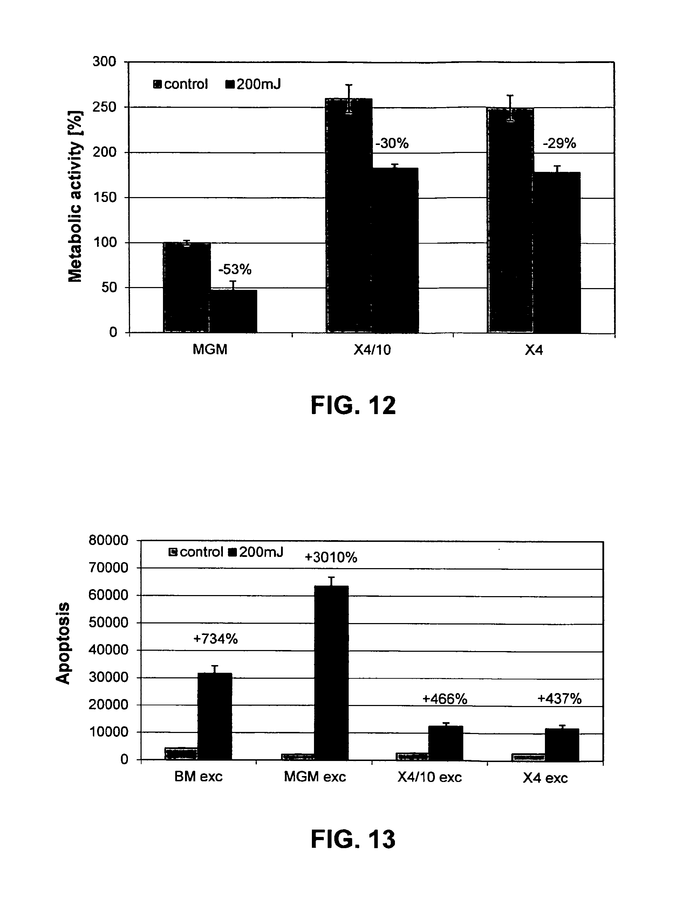

**FIG. 12** is a bar graph showing the metabolic activity 24 h after UV irradiation of NHEK at a UV-B dose of 200 mJ. The NHEK were first incubated in minimal growth medium (MGM), MGM + Pantogar-IC (X4/10) and MGM + Pantogar-IC (X4). Then the respective medium was replaced by MGM, followed by UV-irradiation at a UV-B dose of 200 mJ. The numbers -53%, -30% and -29% indicate the percentage decrease of metabolic activity compared to the metabolic activity of the respective non-UV-irradiated control.
**FIG. 13** is a bar graph showing the degree of apoptosis 14 h after UV irradiation of NHEK at a UV-B dose of 200 mJ. The NHEK were first incubated in basal medium (BM), minimal growth medium (MGM), MGM + Pantogar-IC (X4/10) and MGM + Pantogar-IC (X4). Then the respective medium was replaced by MGM, followed by UV-irradiation at a dose of 200 mJ. The numbers +734%, +3010%, +466% and +437% indicate the percentage increase of apoptosis compared to apoptosis of the respective non-UV-irradiated control.

### DETAILED DESCRIPTION OF THE INVENTION

The present invention is based on the surprising finding that a keratinocyte culture medium that lacks cysteine and/or thiamine mimics limited growth of keratinocytes that is assumed to occur *in vivo* under situations of malnutrition. Based on this discovery, a keratinocyte culture medium was established that can be used in an *in vitro* minimal growth assay system for investigating various aspects of skin physiology.

In particular, the *in vitro* minimal growth assay system finds use as an *in vitro* model that resembles limited growth of human keratinocytes. Since keratinocytes are, in addition to forming the epidermal skin layer, the primary cell type of the hair follicle, limited growth of human keratinocytes is assumed to be associated with diminished hair growth. In particular, limited growth of keratinocyte is assumed to be associated with situations of diffuse hair loss. Thus, the *in vitro* minimal growth assay system allows the identification of compounds that promote growth and proliferation of keratinocytes to thereby obtain candidate compounds for new hair-growth promoting agents.

Furthermore, it was unexpectedly found that the established *in vitro* minimal growth assay system can also be used for investigating the impact of environmental stress, e.g. UV-irradiation. In particular, the system is suitable for identifying compounds capable of protecting keratinocytes from negative effects of UV-irradiation

The novel *in vitro* minimal growth assay system is a simple and efficient means of identifying compounds capable of promoting the proliferation and/or metabolic activity of keratinocytes or protecting keratinocytes from negative effects of UV-irradiation. Since the *in vitro* assessment of metabolic activity, DNA content and apoptosis can be considered as robust surrogate read-out for improvement of the hair-follicle and thus as indirect indicator for enhanced hair growth, the *in vitro* minimal growth assay system of the present invention allows one to screen for candidate compounds promoting hair growth and providing UV protection in a convenient and cost-efficient manner.

Another advantage associated with the *in vitro* model system of the present invention is that the provided in vitro screening system in generally allow to replace, or at least reduce the number of, animal studies. Further, *in vitro* systems are also desirable in view of the fact that, for example for dietary food and moreover for cosmetic compositions, preclinical development is limited in any case to *in vitro* tests due to legal restrictions. Moreover for compounds or compositions with a non-pharmacological mode of action (such as "dietary supplement" or "foods for special medical purpose") it is difficult to identify the suitable study-collective. Either people are close to normal ("healthy") situation, and thus show limited response, or the hair-loss is already pathologic, and thus would need pharmacological intervention.

The term "functional activity", as used herein, refers to any keratinocyte activity that has an impact on the phenotype including, for example, proliferation, metabolic activity, protein expression and/or protein activity, and gene expression. The term "proliferation" refers to cell growth, i.e. the increase of size and volume of single cells, and/or cell division, i.e. the increase in cell number by dividing a parent cell into two or more daughter cells. Within the context of the present invention, the term "proliferation" is generally used to indicate growth of cells or cell populations by cell division.

The term "metabolic activity", as used herein, refers to the sum total of the chemical reactions occurring in keratinocytes measured, for example, by the method described herein below. The term "protein activity", as used herein, refers to any activity of a protein including enzymatic activity, ligand or receptor binding, and the like, which can be assayed using a suitable method, such as a specific enzymatic assay.

The term "expression" is to be understood to refer to the transcription and/or translation of genes, wherein the resulting products are not limited to mRNAs or proteins but may also include functional RNAs such as, for example, antisense nucleic acids, tRNAs, snRNAs, rRNAs, RNAi, siRNA, ribozymes etc., and wherein the gene or protein expression is assessed by measuring the amount or concentration of the respective expression products.

The term "diffuse hair loss", as used herein, refers to a type of hair loss that is seen in both men and women and tends to affect the whole scalp, rather than specific areas of it. Thus, the term "diffuse hair loss" is not androgenetic alopecia or alopecia areata.

The term "improving skin physiology", as used herein, preferably refers to the improvement of natural skin physiology and results in an enhanced function of the skin in terms of providing a protective barrier, reducing water loss, decreasing the number and appearance of skin wrinkles, and the like. Also, it includes anti-ageing effects.

In a first aspect, the present invention relates to a serum-free keratinocyte culture medium, which lacks cysteine and/or thiamin and, optionally, one or more of pantothenate, folic acid and biotin, or derivatives thereof. This culture medium may be derived from known keratinocyte culture media by omitting the mentioned compounds. A particularly suited serum-free keratinocyte culture medium lacks (a) bovine pituitary extract (BPE) and/or epithelial growth factor (EGF) and, optionally, one or more of hydrocortisone, epinephrine, transferrin, or derivatives thereof, and (b) cysteine and/or thiamin and, optionally, one or more of pantothenate, folic acid and biotin, or derivatives thereof.

Preferably, the serum-free keratinocyte culture medium is a medium that lacks any protein growth factor and, optionally, one or more of hydrocortisone, epinephrine and transferrin, or derivatives thereof, and further lacks cysteine and/or thiamin and, optionally, one or more of pantothenate, folic acid and biotin, or derivatives thereof. A particularly preferred serum-free keratinocyte culture medium is a culture medium that lacks bovine pituitary extract, epithelial growth factor, hydrocortisone, epinephrine and transferrin, and further lacks cysteine, thiamin, pantothenate, folic acid and biotin.

The term "growth factor", as used herein, means any factor that shows directly or indirectly a positive impact on the growth or proliferation or metabolic activity of keratinocytes. The term "protein growth factor", as used herein, refers to any growth factor that is a protein, wherein the term "protein" is intended to include peptides. Examples of protein growth factors within the meaning of the present invention are fibroblast growth factor (FGF), e.g. α-FGF and β-FGF, keratinocyte growth factor (FGF-7), platelet-derived growth factor (PDGF) epidermal growth factor (EGF), a vascular endothelial growth factor, tumor growth factor alpha (TNF-alpha), and interleukin 6 (IL-6).

Further preferred serum-free keratinocyte culture media within the present invention are the above described serum-free keratinocyte culture media, which further contain one or more of L-methionine, L-histidine, EGF and BPE. Preferably, the culture medium of the present invention includes L-methionine, L-histidine or both L-methionine and L-histidine.

In another preferred embodiment, the present invention relates to one of the serum-free keratinocyte culture media described above, with the proviso that the culture medium is not a keratinocyte culture medium that lacks cysteine and/or cystine and one or more of L-methionine and L-histidine. In particular, the culture medium is not a keratinocyte culture medium that lacks cysteine and/or cystine and L-methionine, L-histidine, D-pantothenat, EGF and BPE.

The term "derivatives thereof', as used herein, is intended to relate to compounds which are derived from the referred parent compound by chemical modifications or which have a very similar structure and exhibit the same or a substantially equivalent function to that of the referred parent compound. Also included within the term "derivatives thereof" are compounds commonly referred to as pro-drugs, which are inactive precursors of active compounds that become active within the body or cells after undergoing metabolic processes. In particular, the term "derivative of cysteine" includes cystine, wherein the cysteine and the cysteine residues of cystine may be present in the L- or D-enantiomer.

In another aspect, the serum-free keratinocyte culture medium is used for screening for candidate compounds for improving skin and hair physiology or for protecting keratinocytes from negative effects of UV irradiation. In particular, the serum-free keratinocyte culture medium may be used for identifying one or more compounds capable of changing the functional activity of keratinocytes under growth-limiting conditions. Another preferred use is the use for identifying one or more compounds capable of protecting keratinocytes from negative effects of UV irradiation.

A further preferred use is the use of the culture medium according to the present invention for screening for compounds capable of impacting the functional phenotype of keratinocytes, for example promoting the proliferation and/or metabolic activity of keratinocytes, changing gene and/or protein expression, or protecting keratinocytes from negative effects of UV-irradiation. Particularly preferred, the serum-free keratinocyte culture medium is used *in vitro* as minimal growth medium leading to limited growth of keratinocytes, wherein an increased proliferation is indicative of the screened compound's ability to promote functional activity of the skin or hair follicle, in particular improving hair growth and/or reduce hair loss.

In another aspect, the present invention relates to an *in vitro* method for identifying one or more compounds capable of changing the functional activity of keratinocytes, in particular promoting the proliferation and/or metabolic activity. In step (a) of this method, keratinocytes are contacted with the serum-free keratinocyte culture medium as described herein and one or more test compounds.

The contacting of the keratinocytes with the serum-free keratinocyte culture medium and the one or more test compounds may be carried out by contacting the keratinocytes with the serum-free keratinocyte culture medium as described herein containing the one or more test compounds, or contacting the keratinocytes with the serum-free keratinocyte culture medium as described herein, followed by the addition of the one or more test compound.

In step (b), the functional activity of the keratinocytes at a particular time after contacting the keratinocytes with the serum-free keratinocyte culture medium, e.g. after 1 to 96 or 12 to 96 hours, preferably 48 to 72 hours, of incubation, is determined and, in step (c), the capability of the one or more test compounds to change the functional activity of the keratinocytes is assessed. Preferably, the functional activity is determined by measuring the metabolic activity and/or the total amount of DNA.

In accordance with a preferred embodiment, step (a) of the method of the invention comprises contacting keratinocytes with the serum-free keratinocyte culture medium described herein and one or more test compounds and, as a control, with the serum-free keratinocyte culture medium as described herein with no test compound(s) added therein. Then, step (b) of determining the functional activity of the keratinocytes at a particular time after contacting the keratinocytes with the serum-free keratinocyte culture medium is conducted. Thereafter, the capability of the one or more test compounds to change the functional activity of the keratinocytes is assed in step (c) by comparing the functional activity of keratinocytes cultured in the culture medium with the one or more test compounds added therein to that of the keratinocytes cultured in the culture medium with no test compound(s) added therein.

Suitable methods for determining the measure of the number of keratinocytes include methods for measuring the metabolic activity and the total amount of DNA as well as direct counting of cells. Preferably, the functional activity is determined by measuring the metabolic activity and/or the total amount of DNA. The metabolic activity can be, for example, detected by a resazurin assay and total amount of DNA as a measure of proliferation can be, for example, detected using a CyQuant^{®} Direct Cell Proliferation Assay kit (Invitrogen).

Prior to step (a), the keratinocytes are usually contacted with and cultured in a growth medium containing growth factors and/or other growth-promoting compounds for a certain period of time, typically 10 to 20 hours, in particular 10 to 15 hours. Then, the growth medium is removed and the keratinocytes are contacted in step (a) with the serum-free keratinocyte culture medium described herein and one or more test compounds.

In accordance with the present invention, the *in vitro* method for identifying one or more compounds capable of changing the functional activity of keratinocytes under growth-limiting conditions can be used for quantifying one or more compound's capability of promoting proliferation and/or metabolic activity of keratinocytes or wherein the method is used for analyzing compositions for the presence of one or more compounds capable of promoting proliferation and/or metabolic activity of keratinocytes.

In yet another aspect, the present invention relates to an *in vitro* method for identifying one or more compounds capable of protecting keratinocytes from negative effects of UV-irradiation. This method comprises steps (a) to (d), wherein step (a) involves contacting keratinocytes with the serum-free keratinocyte culture medium described herein and one or more test compounds. In step (b), the keratinocytes are irradiated with a certain sublethal dose of UV radiation after contacting the keratinocytes with the serum-free keratinocyte culture medium for a particular time. Next, in step (c), a functional activity of the keratinocytes at a particular time after completion of the UV irradiation is determined. Then, in step (d), the capacity of the one or more test compounds to change the functional activity of the keratinocytes is assessed to thereby identify one or more compounds capable of protecting keratinocytes from negative effects of UV-irradiation. Preferably, the functional activity is assessed by analyzing metabolic activity and/or apoptosis.

The contacting of the keratinocytes with the serum-free keratinocyte culture medium and the one or more test compounds may be carried out by contacting the keratinocytes with the serum-free keratinocyte culture medium as described herein containing the one or more test compounds, or contacting the keratinocytes with the serum-free keratinocyte culture medium as described herein, followed by the addition of the one or more test compound.

In a preferred embodiment, the *in vitro* method for identifying one or more compounds capable of protecting keratinocytes from negative effects of UV irradiation comprises contacting keratinocytes with the serum-free keratinocyte culture medium as described herein and one or more test compounds and, as a control, with the serum-free keratinocyte culture medium as described herein with no test compound(s) added therein (step (a)). Then, the keratinocytes are irradiated with a certain sublethal dose of UV radiation after contacting the keratinocytes with the serum-free keratinocyte culture medium for a particular time (step (b)). Next, the functional activity of the keratinocytes is determined at a particular time after completion of the UV irradiation (step (c)).

Finally, the capacity of the one or more test compounds to protect keratinocytes from negative effects of UV-irradiation is assessed by comparing (i) the change in functional activity of keratinocytes cultured in the UV-irradiated culture medium with the one or more test compounds added therein relative to the change in functional activity of keratinocytes cultured in the same but non-UV-irradiated culture medium with (ii) the change in functional activity of keratinocytes cultured in an UV-irradiated culture medium with no test compound(s) added therein relative to the change in functional activity of keratinocytes cultured in the same but non-UV-irradiated culture medium (step (d)).

The irradiation of the keratinocytes with UV-radiation in step (b) preferably starts 0 to 96 hours, more preferably 12 to 48 hours, and most preferably 24 to 48 hours after contacting the keratinocytes with the serum-free keratinocyte culture medium. Furthermore, the keratinocytes are preferably irradiated with UV-radiation in a way that the total sublethal UV-B (290 nm to 315 nm) is in the range of 50 mJ to 600 mJ, preferably from 100 mJ to 350 mJ. Typically, irradiation is carried out for a period of time of from 1 to 60 minutes, in particular from 5 to 30 minutes, and especially preferred from 10 to 20 minutes.

Preferably, the functional activity of the keratinocytes is determined 1 to 96 hours, more preferably 5 to 48 hours, most preferably 14 to 24 hours after UV irradiation. Furthermore, the functional activity is typically determined by measuring the metabolic activity, proliferation (e.g. indirect by quantifying the contant of DNA), or apoptosis. Measuring the metabolic activity and/or apoptosis is preferred within the context of the present invention.

Prior to step (a), the keratinocytes are usually contacted with and cultured in a growth medium containing growth factors and/or other growth-promoting compounds for a certain period of time, typically 10 to 20 hours, in particular 10 to 15 hours. Then, the growth medium is removed and the keratinocytes are contacted in step (a) with the serum-free keratinocyte culture medium described herein and one or more test compounds.

In accordance with the present invention, the *in vitro* method for identifying one or more compounds capable of protecting keratinocytes from UV radiation can be used for quantifying the capacity of the one or more compounds to protect keratinocytes from negative effects of UV irradiation, or for analyzing compositions for the presence of compounds capable of protecting keratinocytes from negative effects of UV irradiation.

In a yet further aspect, the present invention relates to a kit, which comprises the serum-free keratinocyte culture medium of the present invention.

The present invention will now be further illustrated by the following examples.

### EXAMPLES

The following examples shows that the novel *in vitro* "minimal growth" assay system of the present invention mimics limited growth of keratinocytes and that this assay is suited for use as an *in vitro* model for studying the effects of compounds on growth and activity of keratinocytes under limited growth conditions. This capacity of the compound is expected to translate into improvements of skin physiology and particularly in improved hair-follicle activity and, thus, finally improved hair growth. In particular, the novel *in vitro* system allows compounds to be identified that are capable of promoting the functional capacity of keratinocytes as assessed e.g. by increased proliferation and/or metabolic activity. Such compounds are potential candidates for promising new skin and hair-growth promoting agents.

The following culture media and test methods were used in the Examples provided below:

### (1) Culture media

"BM" (Basal Medium): serum-free keratinocyte media (as provided in the "Keratinocyte Growth medium 2"-Kit available from PromoCell), without bovine pituitary extract (BPE), epidermal growth factor (EGF), hydrocortisone, epinephrine, and transferrin.

"GM" (Growth Medium): supplemented BM, including bovine pituitary extract (BPE), epidermal growth factor (EGF), hydrocortisone, epinephrine, and transferrin ("Keratinocyte Growth medium 2 ready to use", available from PromoCell).

"MGM" (Minimal Growth Medium according to the present invention): BM without cysteine, thiamine, pantothenate, folic acid, and biotine.

All media used have a calcium concentration of 60 µM and are serum-free.

### (2) Test methods

Metabolic activity was measured by a resazurin assay. In this assay, non-fluorescent resazurin is reduced to fluorescent resorufin in viable cells only. The resulting signal is proportional to the number of cells present and, thus, is an indirect measure of cell number.

The assay was carried out by diluting a resazurin (Sigma) stock solution (0,1 mg/ml in PBS) in the respective culture medium at a ratio of 1:11. The resazurin conversion to the fluorescent resorufin by metabolically active cells was recorded at 560_{Ex}/590_{Em} nm using a Synergy^{™} H4 plate reader (BioTek Instruments).

Alternatively, cell number was directly assessed by measuring the amount of DNA using the CyQuant^{®} Direct Cell Proliferation Assay kit (Invitrogen). In brief, the two CyQuant^{®} kit solutions were mixed and added to the respective culture medium as described by the supplier. The fluorescence of the DNA intercalating CyQuant^{®} Dye, representing the amount of cells, was recorded at 480_{Ex}/535_{Em} nm using a Synergy^{™} H4 plate reader (BioTek Instruments). Increase of the number of cells is used as indirect indicator of cell proliferation.

Apoptosis was analysed using the Caspase Glo 3/7 Assay (Promega). The reconstituted Caspase Glow 3/7 reagent was added to the cells and incubated as described by supplier. In this assay, a substrate is specifically cleaved by the endogenous Caspase 3/7 activity of NHEK and in addition consumed by a thermostable luciferase, generating a "glow-type" luminescent signal. 14 h after UV-irradiation this luminescence signal representing the amount of apoptotic cells was measured using a Synergy^{™} H4 plate reader (BioTek Instruments).

Mean values of relative fluorescence/luminescence units (RFU/RLU) and standard deviation of replicates were analysed by Gene5 software. For a better estimation of test compound's potential metabolic activity and proliferation measured in MGM were set as 100%. Statistical significance was assessed by comparing mean (+/-SD) values with Student's t-test for independent groups.

### Example 1

### Establishment of "minimal growth" assay system

NHEK (normal human epidermal keratinocytes) were seeded at 30,000 cells/cm² into 48-well flat bottom tissue culture plates and cultured over night (typically 12 to 15 h) in growth medium (GM) at 37°C and 5% CO₂. The growth medium was then replaced by either minimal growth medium (MGM), basal medium (BM) or again growth medium (GM) and cultivation was continued at 37°C and 5% CO₂.

Next, 24, 48 and 72 hours after the medium replacement, the impact of the changed culture condition on the cell population was characterized by analyzing the cell number indirectly via metabolic activity using the resazurin assay (see FIG. 1; the metabolic activity data are shown relative to that of the MGM after 24 h, which was set 100%) and directly by detection of the DNA amount using the CyQuant^{®} Direct Cell Proliferation Assay kit (results not shown). MGM-cultivation mediated reduced cell number was also confirmed by microscopic analysis (data not shown).

As can be seen from FIG. 1, cultivation of keratinocytes in MGM resulted in reduced value for metabolic activity resembling limited activity of keratinocytes during malnutrition in which the cells are still capable, although on a greatly reduced basis, of metabolic activity and/or proliferation. Thus, this *in vitro* minimal growth assay system using MGM imitates limited growth of keratinocytes, such as that associate with diminished hair-growth.

In other words, the cultivation in MGM represents an *in vitro* model for undersupplied keratinocytes that can be used as model for situations of diminished hair growth.

### Example 2

### Metabolic activity as surrogate for cell number

This Example shows that metabolic activity can be used as a measure of the number of cells in all three tested culture media.

NHEK were seeded into minimal growth medium (MGM), basal medium (BM) and growth medium (GM) and cultivated. The metabolic activity was measured for the three different media using the resazurin assay. The results are shown in **FIG. 2** and **FIG. 3****.**

From **FIG. 2** and **FIG. 3**, it can be seen that the used assays shows a linear correlation between measured fluorescence intensity and cell number. Accordingly, metabolic activity is a surrogate for cell number.

### Example 3

### Evaluation of the capability of L-cystine, thiamine, Ca²⁺-pantothenate

### and folic acid to promote the growth of keratinocytes

In this example, the effect of four different ingredients included in Pantogar (i.e. L-cystine, thiamine, Ca²⁺-pantothenate and folic acid) on metabolic activity and proliferation in NHEK were tested in a dose-dependent manner as single compounds and as compositions.

### 1. Cultivation of human epidermal keratinocytes (NHEK) in normal growth medium

NHEK were seeded at 30.000 cells/cm² into 48-well flat bottom tissue culture plates and cultured in normal keratinocyte growth medium ("GM") at 37°C and 5% CO₂.

### 2. Replacement of the growth culture medium by the minimal growth medium

After 12 to 15 h (typically over night) the media was exchanged to MGM with different concentrations of test compound. Controls receive MGM without compounds.

Test compounds 1 to 4 (1: L-cystine; 2: thiamine; 3: Ca²⁺-pantothenate; 4: folic acid) were prepared according to Table 1 to yield respective stock solutions. The stock solutions were further diluted in minimal growth media (MGM) to obtain different test compound concentrations. The final Cₘₐₓ in the assay was 1 mM for thiamin, 0.5 mM for L-cystine and 0.1 mM for pantothenate and folic acid. The negative control wells contained media only plus the respective solvent.

**Table 1. Test compound and control solutions**

| No. | Name | Solvent | Final [mM] |
|---|---|---|---|
| 1 | L-cystine | MGM | 0,5 |
| 2 | Thiamin | DMSO | 100 |
| 3 | Ca²⁺-pantothenate | DMSO | 100 |
| 4 | Folic acid | DMSO | 100 |

### 3. Measurement of metabolic activity and proliferation

For measurements of metabolic activity and proliferation, NHEK were seeded at 30,000 cells/cm² into a 48-well microtiter flat bottom plate. After over night incubation growth medium 2 was replaced by BM, MGM, and MGM plus test compounds. The test compounds were dissolved in DMSO, diluted into MGM and added to the cells. 4 to 6 replicates/concentration were tested. Each plate included 2 controls (BM and MGM alone, 4 to 6 replicates), a compound interference control (all concentrations, duplicates) and a background control (2 replicates).

Metabolic activity after 48 h (see **FIG. 4**) and 72 h after incubation (see **FIG. 5**) was determined by the resazurin assay, and proliferation after 48 h and 72 h after incubation was measured using the CyQuant^{®} Direct Cell Proliferation Assay kit (data not shown). In **FIGS. 4-5**, the metabolic activity is shown relative to that of the MGM control, which was set 100%.

As a result, it was found that L-cystin demonstrates a marked dose-depending increase of metabolic activity/cell number up to 265% after 48 h (see **FIG. 4(a)**) and up to 419% after 72 h (see **FIG. 5(a)**) compared to the MGM control (only minimal growth medium without L-cystin). Thiamin also exerts a significant effect in that it clearly leads to a dose-depending increase of metabolic activity/cell number of up to 138% after 48 h (see **FIG. 4(b)**) and up to 163% after 72 h (see **FIG. 5(b)**) compared to the MGM control. The greatest effects are observed at high concentrations of thiamine.

In contrast, pantothenate did not show a dose-depending increase of metabolic activity/cell number compared to the MGM control. For example, 5 µm pantothenate resulted in a metabolic activity of 111% after 48 h (see **FIG. 4(c)**), and 100 µm pantothenate resulted in a metabolic activity of 110% after 72 h (see **FIG. 5(c)**) compared to the MGM control. Likewise, folic acid did not demonstrate a dose-depending increase of metabolic activity/cell number compared to the MGM control. For example, 100 µm folic acid resulted in a metabolic activity of 113% after 48 h (see **FIG. 4(d)**) and 118% after 72 h (see **FIG. 5(d)**) compared to the MGM control.

### Example 4

### Impact of Pantogar-IC on metabolic activity and proliferation

This example aims at determining whether a combination of L-cystine, thiamine, pantothenate and folic acid as a potential *in vitro* correlate to the pantogar formulation ("Pantogar-IC") is superior over the effects of these compounds alone.

The data presented in **FIG. 6** demonstrate that the combination is superior compared to the effect of the single compounds. MGM basal values could be increased to > 440% after 72 h. The greatest effects were observed at X4/10. Saturation may occur from X4/30 on with incubations ≥ 72 h. These observations were also confirmed by measuring the DNA content after 72 h instead of measuring the metabolic activity after 72 h (data not shown).

In sum, it was found that the established combination of L-cystine, thiamine, pantothenate and folic acid was able to restore the minimal proliferation status back to "normal", i.e. basal media values. The Pantogar-IC is therefore indeed an *in vitro* correlate to the pantogar formulation. Therefore, in the established *in vitro* model for undersupplied keratinocytes, Pantogar is able to restore the metabolic activity to normal growth conditions.

### Example 5

### Synergistic effects of L-cystine, pantothenate and folic acid

As demonstrated in Example 3, the single addition of calcium pantothenate and folic acid, respectively, resulted in essentially no increase in metabolic acitivity compared to the MGM control (see **FIG. 4** and **FIG. 5**).

However, it was unexpectedly found that the addition of calcium pantothenate (CaP) to L-cystine (Cys) increases proliferation, measured by quantifying the DNA amount using the CyQuant^{®} Direct Cell Proliferation Assay kit, from 282% up to 305% (see **FIG. 7**). Furthermore, the addition of both calcium pantothenate (CaP) and folic acid (FS) (a potential prokaryotic PABA metabolite) to L-cystine was found to result in an increased proliferation of 383% compared to the MGM control (see **FIG. 7**).

Thus, it was established that L-cysteine in combination with calcium pantothenate alone or in combination with both calcium pantothenate and folic acid surprisingly exerts an increased proliferative effect on keratinocytes compared to L-cysteine alone.

### Example 6

### Impact of UV-radiation on the metabolic activity and proliferation of

### normal human epidermal keratinocytes (NHEK)

In this example, the in vitro "minimal growth" assay system according to the present invention was used to investigate the impact of UV-radiation (UVR) as an example of environmental stress.

UVR has a known major impact on the skin. In addition to its inhibitory effect on epidermal keratinocyte proliferation and induction of apoptosis, UVR promotes skin aging and carcinogenesis. A damaging effect of UVR on the hair has been described for the extracutaneous part of the human hair shaft *in vivo* and also *in vitro* on the cellular level for organ-cultured hair follicles.

NHEK were seeded at 30,000 cells/cm² into a 48-well microtiter flat bottom plate. After over night incubation growth medium (GM) was replaced by BM, MGM, and MGM plus test compounds. The test compounds were dissolved in DMSO (Roth), diluted into MGM and added to the cells. 4 replicates/concentration were tested. Each plate included 1 to 2 controls (BM and MGM alone, 4 replicates), a compound interference control (all concentrations, duplicates) and a background control (2 replicates).

24 h after incubation under indicated conditions cells were irradiated using a solar-simulator (290 to 800 nm, Suntest CPS). Control cells were kept shielded in the solar simulator to avoid irradiation. Metabolic activity was determined 24 h after UV-irradiation by recording the fluorescence intensity of metabolized resazurin at 560_{Ex}/590_{Em} nm, as described in Example 1.

NHEKs were cultivated in minimal growth medium (MGM) as described in Example 3 and irradiated with UV-B doses varying from 50 mJ to 600 mJ. The UVR (290-400nm) mimicked UVR as found on earth surface and was generated using a solar-simulator (Atlas Suntest CPS/CPS+). The results shown in **FIG. 8** demonstrate that the metabolic activity decreases in a UVR-dose dependent manner. For the following experiments, an UV-B irradiation dose of 200 mJ (reduction of control activity to < 40%) was chosen.

### Example 7

### UV-induced apoptosis of normal human epidermal keratinocytes (NHEK)

Apoptosis was analysed using the Caspase Glo 3/7 Assay (Promega). The reconstituted Caspase Glow 3/7 reagent was added to the cells and incubated as described by supplier. In this assay, a substrate is specifically cleaved by the endogenous Caspase 3/7 activity and in addition consumed by a thermostable luciferase, generating a "glow-type" luminescent signal. 14 h after UV-irradiation this luminescence signal representing the amount of apoptotic cells was measured using a Synergy^{™} H4 plate reader (BioTek Instruments).

As can be seen from **FIG. 9**, the maximum apoptosis signal was measured 14 h post UV irradiation. Thus, an irradiation time of 14 h was chosen for the following experiments.

### Example 8

### UV-protective effect of Pantogar on normal human epidermal keratinocytes (NHEK)

The UV-protective effect of the *in vitro* correlate to the Pantogar formulation (Pantogar-IC), consisting of 0.5 mM L-cystine, 1 mM thiamine, 10 µM pantothenate and 10 µM folic acid, was assessed by cultivating NHEK in minimal growth medium (MGM), MGM + X4/10, and MGM + X4 (X4 = Pantogar-IC) with UVR (200 mJ) or without UVR (control), and measuring the metabolic activity after 24 h.

As can be seen from **FIG. 10**, the metabolic activity of the UV-radiated NHEK in MGM is reduced by 72% compared to the non-radiated control. The addition of Pantogar-IC to MGM resulted in a decrease of metabolic activity, as compared to the control (non-radiated MGM), of only 12% and 18% for X4/10 and X4, respectively. Thus, these results show that Pantogar-IC is UV-protective.

Furthermore, the same experiment as that described above was carried out, except that apoptosis was measured instead of metabolic activity. The results are shown in **FIG. 11**. As a result, it was found that Pantogar-IC drastically decreased the apoptotic rate of keratinocytes compared to MGM. Further the UVR induced apoptotic rate of keratinocytes was found to be reduced in a dose-dependent manner.

### Example 9

### Mode of action of Pantogar mediated UV-protection

Further experiments were carried out to find out whether the UV-protective effect of the Pantogar-IC is simply due to "UV filtering" effects or due to "metabolic" effects supporting cellular defense mechanisms.

The UV-filter effect and the metabolic protection effect were dissected by replacing MGM + Pantogar-IC (i.e., X4/10 or X4) by MGM only during irradiation to eliminate a potential UV-filtering effect. The UV-protective effect of Pantogar-IC was assessed by measuring the metabolic activity 24h after UV irradiation (see **FIG. 12**) and the apoptosis induction 14 h after UV irradiation (see **FIG. 13**).

From **FIG. 12** and **FIG. 13****,** it can be seen that Pantogar-IC exerts an UV-protective effect even when replaced during irradiation (i.e. omitting a UV-filtering effect). Interestingly, Pantogar-IC/10 (X4/10) is equally effective as Pantogar-IC (X4), indicating that the UV-protective effect is not depending on UV-filtering effects. In view of these results, it appears that the Pantogar-mediated UV-protection is not just based on UVR-filtering but, surprisingly, mainly mediated via "metabolic" support or "metabolic" reinforcement of NHEKs.

## Claims

1. A serum-free keratinocyte culture medium, wherein the culture medium lacks cysteine and/or thiamin and, optionally, one or more of pantothenate, folic acid and biotin, or derivatives thereof.

2. The serum-free keratinocyte culture medium of claim 1, wherein the culture medium lacks (a) bovine pituitary extract and/or epithelial growth factor and, optionally, one or more of hydrocortisone, epinephrine and transferrin, or derivatives thereof, and (b) cysteine and/or thiamin and, optionally, one or more of pantothenate, folic acid and biotin, or derivatives thereof.

3. The serum-free keratinocyte culture medium of claim 1 or 2, wherein the culture medium lacks bovine pituitary extract, epithelial growth factor, hydrocortisone, epinephrine and transferrin, and further lacks cysteine, thiamin, pantothenate, folic acid and biotin.

4. The serum-free keratinocyte culture medium of any one of claims 1 to 3, which is not a serum-free keratinocyte culture medium that lacks cysteine and/or cystine and one or more of L-methionine and L-histidine.

5. Use of the serum-free keratinocyte culture medium of any one of claim 1 to 4 for screening for candidate compounds for improving skin and hair physiology or for protecting keratinocytes from negative effects of UV irradiation.

6. An *in vitro* method for identifying one or more compounds capable of changing the functional activity of keratinocytes under growth-limiting conditions, the method comprising:
(a) contacting keratinocytes with the serum-free keratinocyte culture medium according to any one of claims 1 to 4 and one or more test compounds;
(b) determining the functional activity of the keratinocytes at a particular time after contacting the keratinocytes with the serum-free keratinocyte culture medium;
(c) assessing the capability of the one or more test compounds to change the functional activity of the keratinocytes.

7. The method of claim 6, wherein the capability of the one or more test compounds to change the functional activity of the keratinocytes is assessed by comparing the functional activity of keratinocytes cultured in the culture medium with the one or more test compounds added therein to that of the keratinocytes cultured in the culture medium with no test compound(s) added therein.

8. The method of claim 6 or 7, wherein, in step (b), the functional activity is determined 12 to 96 hours after contacting the keratinocytes with the serum-free keratinocyte culture medium and/or wherein the functional activity is determined by measuring the metabolic activity and/or the total amount of DNA.

9. The method of any one of claims 6 to 8, wherein the method is used for quantifying one or more compound's capability of promoting proliferation and/or metabolic activity of keratinocytes or wherein the method is used for analyzing compositions for the presence of one or more compounds capable of promoting proliferation and/or metabolic activity of keratinocytes.

10. An *in vitro* method for identifying one or more compounds capable of protecting keratinocytes from negative effects of UV irradiation, the method comprising:
(a) contacting keratinocytes with the serum-free keratinocyte culture medium according to any one of claims 1 to 4 and one or more test compounds;
(b) irradiating the keratinocytes with a certain sublethal dose of UV radiation after contacting the keratinocytes with the serum-free keratinocyte culture medium for a particular time;
(c) determining a functional activity of the keratinocytes at a particular time after completion of the UV irradiation; and
(d) assessing the capacity of the one or more test compounds to change the functional activity of the keratinocytes to thereby identify one or more compounds capable of protecting keratinocytes from negative effects of UV radiation.

11. The method of claim 10, wherein the capacity of the one or more test compounds to change the functional activity of the keratinocytes is assessed by comparing (i) the change in functional activity of keratinocytes cultured in the UV-irradiated culture medium with the one or more test compounds added therein relative to the change in functional activity of keratinocytes cultured in the same but non-UV-irradiated culture medium with (ii) the change in functional activity of keratinocytes cultured in an UV-irradiated culture medium with no test compound(s) added therein relative to the change in functional activity of keratinocytes cultured in the same but non-UV-irradiated culture medium.

12. The method of claim 10 or 11, wherein, in step (b), the exposure of the keratinocytes to UV radiation begins 12 to 48 hours after contacting the keratinocytes with the serum-free keratinocyte culture medium and/or wherein the sublethal UV dose is an UV-B dose of 50 mJ to 600 mJ.

13. The method of any one of claims 6 to 12, wherein, prior to step (a), the keratinocytes are contacted with and cultured in a growth medium containing growth factors and/or other growth-promoting compounds for a certain period of time, followed by removal of the growth medium.

14. The method of any one of claims 10 to 13, wherein the method is used for quantifying the capacity of the one or more compounds to protect keratinocytes from negative effects of UV irradiation, or wherein the method is used for analyzing compositions for the presence of compounds capable of protecting keratinocytes from negative effects of UV irradiation.

15. A kit, comprising a serum-free keratinocyte culture medium according to any one of claims 1 to 4.
